# EUROPEAN PATENT APPLICATION

(11) **EP 4 599 770 A2**
(43) Date of publication of application: **13.08.2025**
(21) Application number: 25151739.7
(22) Date of filing: 14.01.2025
(51) Int. Cl.: A61B 6/03, A61B 6/00

(54) **FRICTION WHEEL MOTORIZATION FOR A FLOOR MOUNTED MEDICAL IMAGING DEVICE**

(30) Priority: 07.02.2024 US 202418435926
(71) Applicant: GE Precision Healthcare LLC, Waukesha, WI 53188 (US)
(72) Inventor: BOUVIER, Bernard, 78533 Buc (FR); MARTINEZ FERREIRA, Carlos, 78533 Buc (FR); MARIE, Arnaud, 78533 Buc (FR)
(74) Representative: AWA Sweden AB

(57) **Abstract**

Systems and methods are provided for a medical imaging system (200, 10). In one embodiment, a mobile floor support assembly (208) for a medical imaging assembly (204) comprises a base (210) configured to swivel on a swivel axis (304), and a vertical member (212) coupled to the base (210), the vertical member (212) configured to hold the medical imaging assembly (204) above the base (210). The mobile floor support assembly (208) comprises a flat bearing (506) positioned in the base (210), the flat bearing (506) supporting the swivel axis (550, 414, 304), and a motorized friction wheel (508) positioned at a junction (514) of the vertical member (212) and the base (210). The motorized friction wheel (508) controls the swivel on the swivel axis (304).

## Description

### FIELD

Embodiments of the subject matter disclosed herein relate to motorization of a floor mounted C-arm interventional radiology system.

### BACKGROUND

Conventional C-arm interventional radiology is commonly used by medical professionals to provide a range of imaging services including diagnostic applications and interventional applications. For example, C-arm imaging systems may be used for real-time imaging during surgeries, visualizing bone structures and injuries, guiding minimally invasive procedures, and in dentistry for oral surgery, endodontics, and orthodontics. Commonly, C-arm imaging systems are ceiling mounted or floor mounted. Floor mounted C-arm systems generally include a C-arm gantry rotatably mounted to a base, an X-ray tube mounted to the C-arm, and an X-ray detector assembly mounted to the C-arm opposing the X-ray tube.

The C-arm gantry may be moved relative to an imaging subject to obtain X-ray images from a plurality of angles and positions. For example, the C-arm gantry may be rotated and swiveled around the patient, as well as shifted linearly around a procedure room. As such, C-arm imaging systems generally include electromechanical components for controlling a position of the C-arm gantry relative to the imaging subject. Electromechanical components for controlling the position of the C-arm gantry may be bulky and increase the overall size of the device, and for floor mounted C-arm imaging systems, bulky components may take up valuable floor space and get in the way of medical professionals.

### BRIEF DESCRIPTION

In one embodiment, a mobile floor support assembly for a medical imaging assembly comprises a base configured to swivel on a swivel axis, a vertical member coupled to the base, the vertical member configured to hold the medical imaging assembly above the base, a flat bearing positioned in the base, the flat bearing supporting the swivel axis, and a motorized friction wheel positioned at a junction of the vertical member and the base, wherein the motorized friction wheel controls swivel on the swivel axis. In this way, swiveling is achieved without bulky electromechanical components.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The present invention will be better understood from reading the following description of non-limiting embodiments, with reference to the attached drawings, wherein below:
FIG. 1 shows a block diagram of an example of an imaging system;
FIG. 2 shows an imaging system including an example of a floor mounted mobile support assembly for an X-ray machine of the present disclosure;
FIG. 3 shows a diagram depicting spatial considerations related to the X-ray machine of FIG. 2;
FIG. 4 shows a block diagram depicting an approach for controlling swivel movement for an X-ray machine of the present disclosure;
FIG. 5 shows one example of a support structure implementing the approach of FIG. 4 for controlling swivel movement for an X-ray machine;
FIG. 6 shows a section view of an X-ray machine of the present disclosure including a motorized friction wheel and a flat bearing;
FIG. 7 shows a diagram depicting dimensions achieved with the approach for controlling swivel movement for an X-ray machine of the present disclosure;
FIG. 8 shows a first schematic diagram depicting a wheel assembly for the mobile floor support assembly of the present disclosure;
FIG. 9 shows a second schematic diagram of the wheel assembly of FIG. 8; and
FIG. 10 shows an exemplary method for controlling an X-ray machine of the present disclosure.

### DETAILED DESCRIPTION

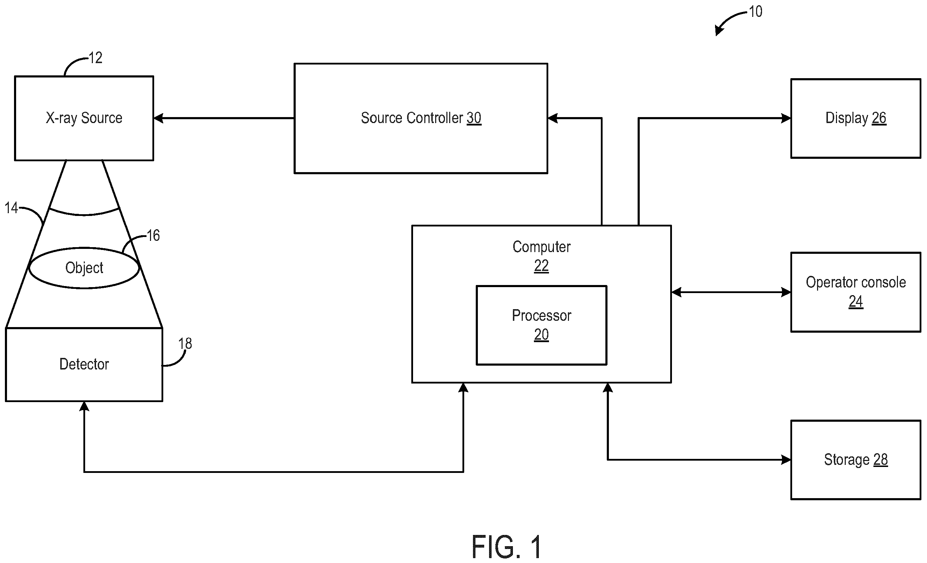

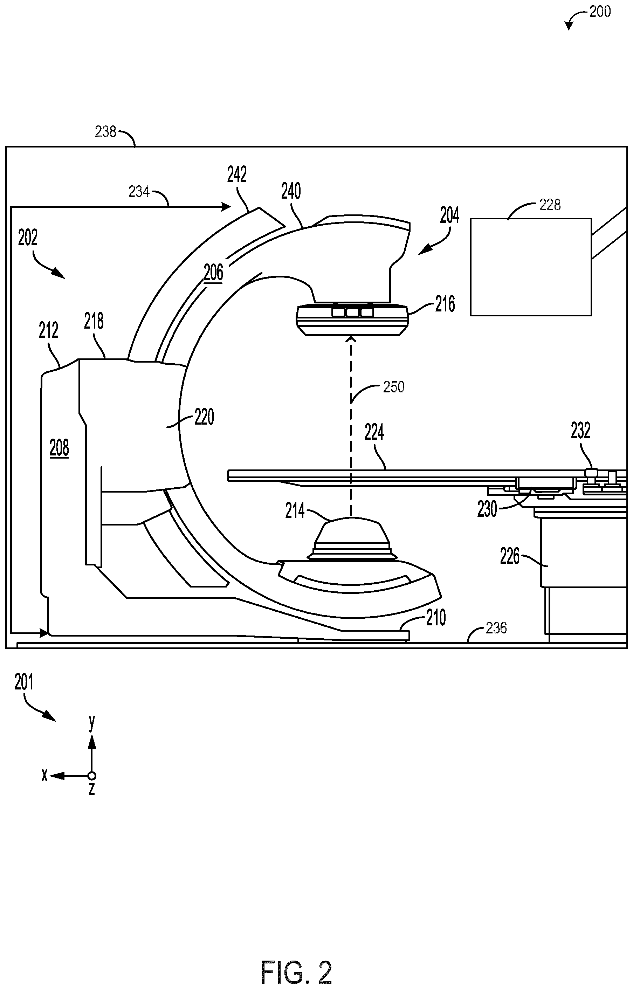

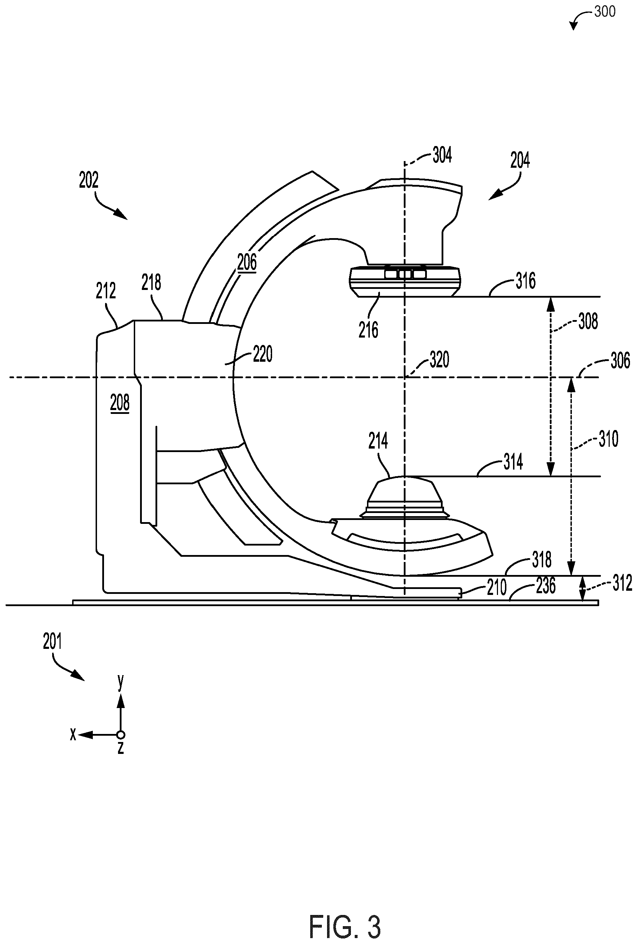

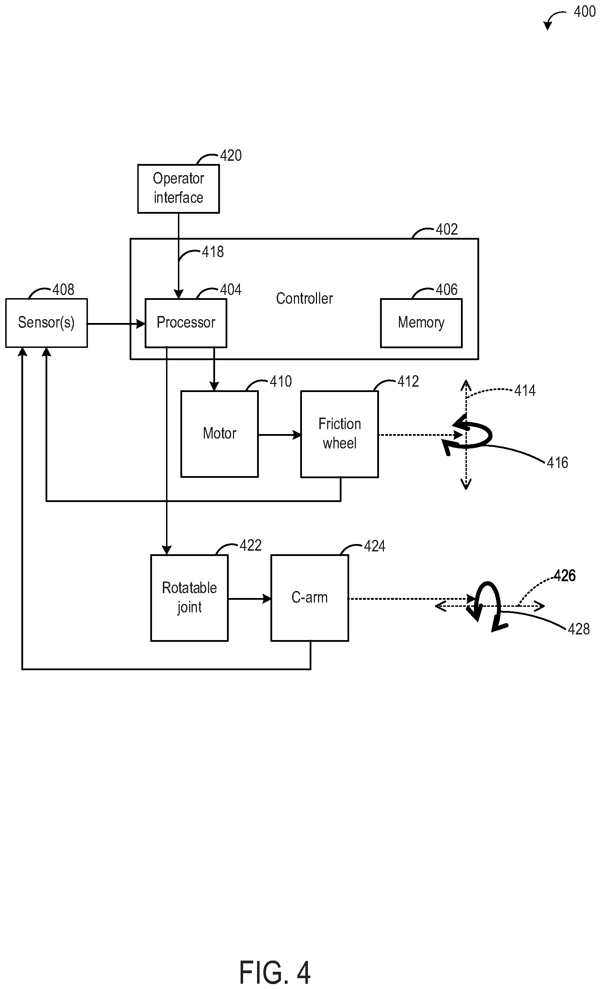

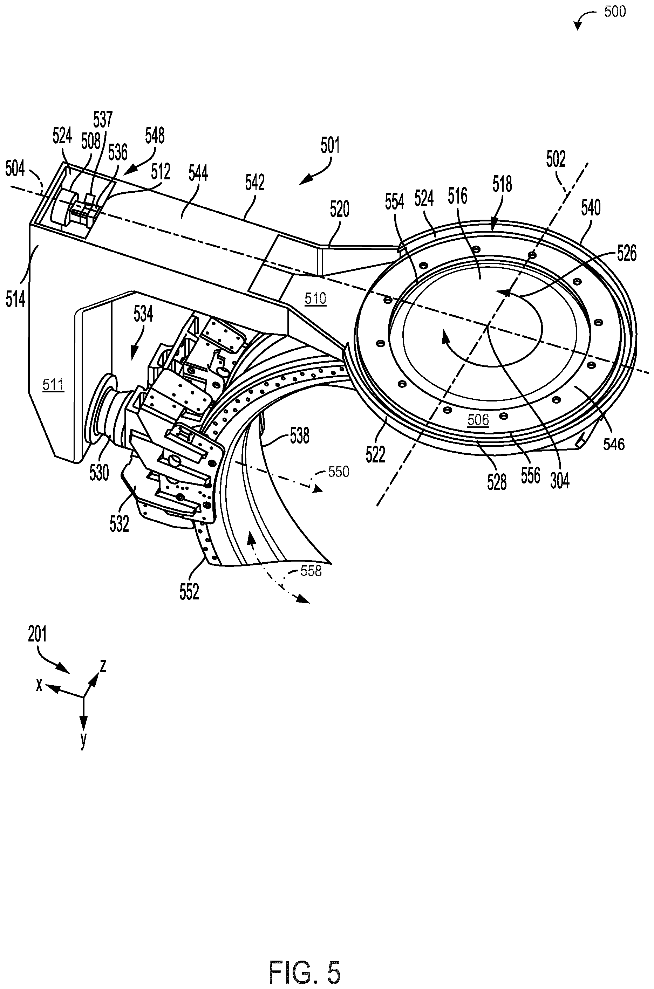

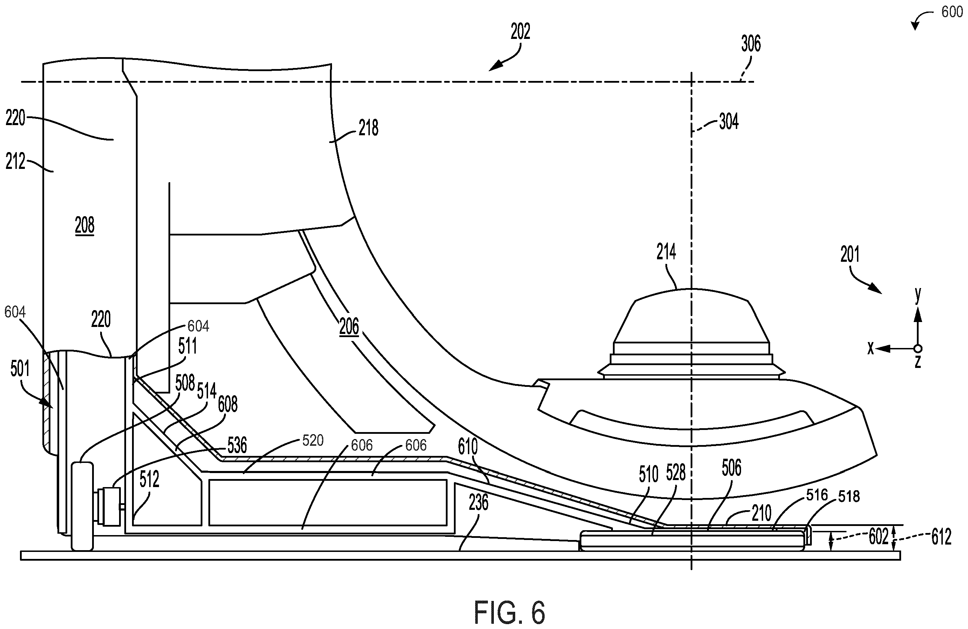

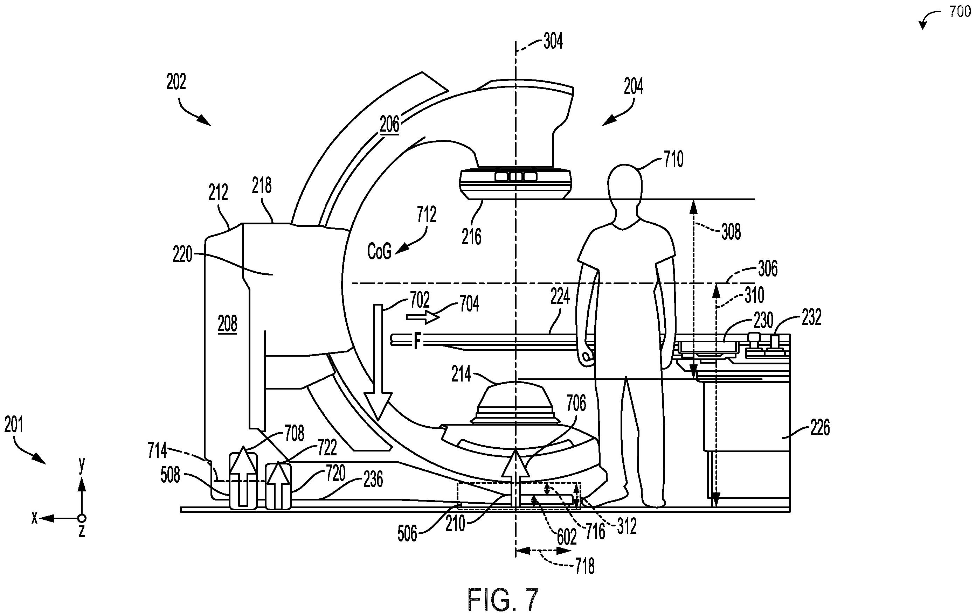

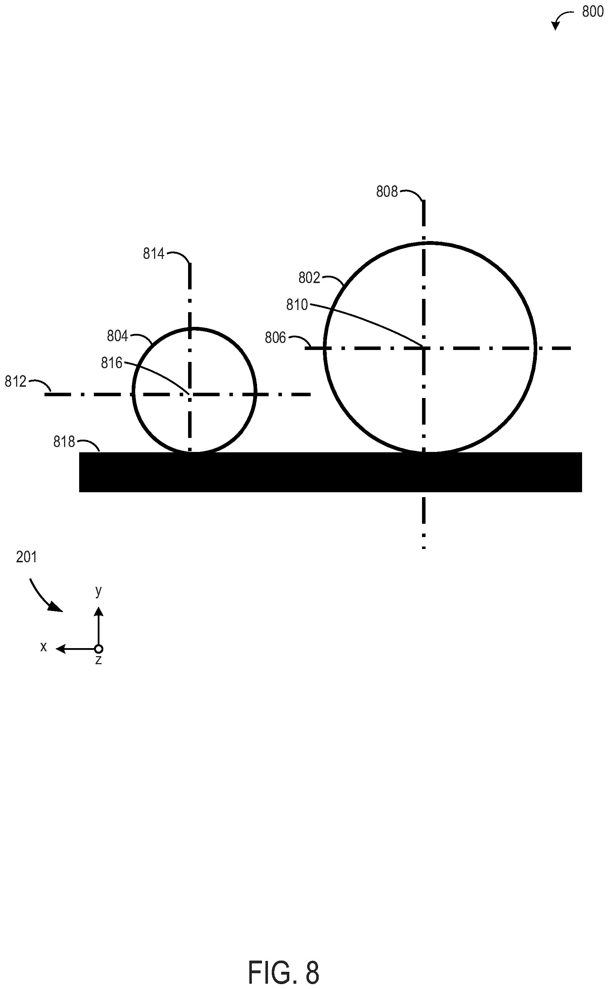

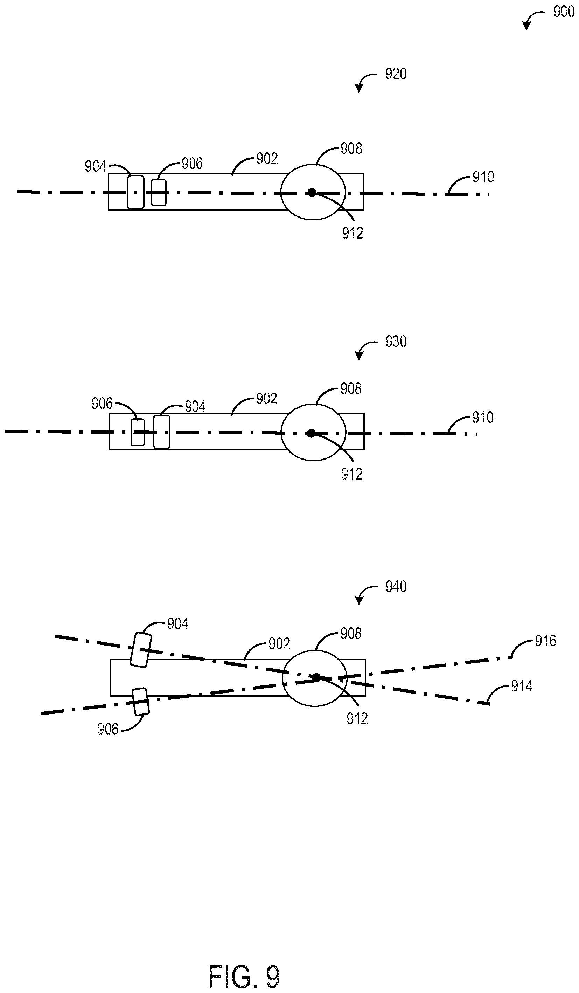

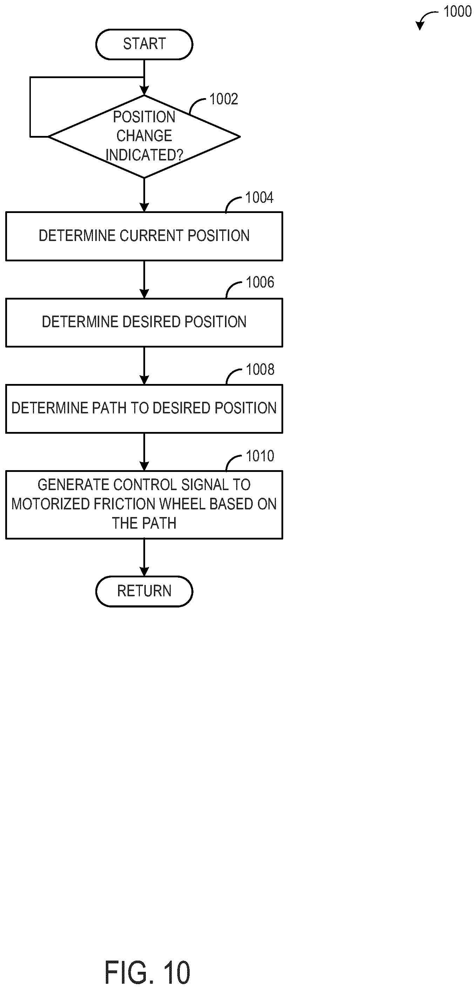

The following description relates to various embodiments for an X-ray machine including systems and methods for a mobile floor support assembly with swivel movement and a large opening for imaging at a comfortable working height. The X-ray machine may be included in an X-ray imaging system, an example block diagram of which is shown in FIG. 1. The X-ray imaging system may be an interventional radiography imaging system, a fluoroscopic imaging system, a mammography imaging system, a fixed or mobile radiography (RAD) imaging system, a tomographic imaging system, a computed tomography (CT) imaging system, and so on. The X-ray imaging system includes an X-ray source (e.g., an X-ray tube) to generate irradiating X-ray beams. An example of an imaging system including a floor mounted C-arm interventional radiology system is shown in FIG. 2. The floor mounted C-arm interventional radiology system is based on centered C-arc technology. Spatial considerations related to the centered C-arc technology are shown in FIG. 3. Among the spatial considerations are constraints related to electromechanical components that control and support swivel movement of C-arm systems, which may influence the size and working height of the C-arm. A block diagram illustrating an approach to control and support movement for a C-arm system of the present disclosure is shown in FIG. 4. One example of the mobile floor support assembly of the present disclosure, including a motorized friction wheel controlling a swivel axis, is shown in FIG. 5. FIG. 6 and FIG. 7 show dimensions achieved with the mobile floor support assembly of the present disclosure. The disclosed X-ray machine may include a free wheel that is driven by the motorized friction wheel. A block diagram of a wheel assembly is shown in FIG. 8. A second diagram of the wheel assembly is shown in FIG. 9. An exemplary method for controlling an X-ray machine of the present disclosure is shown in FIG. 10.

C-arm interventional radiology systems, also referred to as CT scanner systems, may obtain X-ray images from a plurality of angles and positions. C-arm imaging systems generally comprise a C-arm gantry, an X-ray tube mounted to the C-arm gantry, and an X-ray detector assembly mounted to the C-arm gantry opposing the X-ray tube. The C-arm gantry is moveably mounted to base. For example, the C-arm gantry may rotate and swivel relative to the base. In some examples, the base is L-shaped, and may be referred to as an L-arm. Commonly, C-arm CT imaging systems are ceiling mounted or floor mounted. Floor mounted C-arm imaging systems generally include a base that rests on the floor and a vertical member to which the C-arm is rotatably mounted. Swivel and rotational movement of the C-arm gantry may be achieved by operation of electromechanical components, which in floor mounted C-arm CT systems may be housed in one or more of the vertical member of the L-arm, the base of the L-arm, and the C-arm gantry. For example, swivel movement of floor mounted C-arm imaging systems may include electromechanical components housed in the base.

C-arm imaging systems may include centered C-arc or eccentric C-arc designs. For example, eccentric designs may include the C-arm gantry mounted to the L-arm where a rotational axis of the C-arm is offset from a vertical axis of the L-arm. Centered C-arc designs may include the C-arm gantry mounted to the L-arm centered on a vertical plane that divides the C-arm imaging system laterally into substantially equal halves. While the centered configuration provides flexibility in the position of a medical professional to perform different procedures, there are also size and height considerations that constrain centered C-arc designs. For example, there may be a height range within which a medical professional may comfortably operate the C-arm imaging system. For one non-limiting example, a comfortable operating height may be relative to a person of average size for a typical work flow. Further, there may be a minimum distance between the X-ray tube and the X-ray detector, also called a 3D tunnel, within which high-quality, high-detail X-ray images are obtained for an imaging subject. Further, there may be a minimum distance between the X-ray tube and the detector within which patients may comfortably fit for imaging.

Additional bulk or base height that increases the height of the C-arm or reduces the distance between the X-ray tube and the X-ray detector may be undesirable. For example, swivel movement of floor mounted C-arm imaging systems may include a chain driven motor and slewing bearing type. Such configurations may be bulky and result in tradeoffs between achieving a comfortable working height and the size of the 3D tunnel. For example, some designs may reduce the size of the 3D tunnel to achieve a comfortable working height, and other designs may maintain a large 3D tunnel at a cost to operator comfort. A floor mounted C-arm design that achieves a large 3D tunnel and a comfortable working height is desired.

Thus, a mobile floor support assembly for a medical imaging assembly is disclosed. In one embodiment, the mobile floor support assembly comprises a base configured to swivel on a swivel axis and a vertical member coupled to the base. The vertical member is configured to hold the medical imaging assembly above the base. A flat bearing, which supports the swivel axis, is positioned in the base and a motorized friction wheel is positioned at a junction of the vertical member and the base. The motorized friction wheel may be controlled to swivel on the swivel axis. In one example, the flat bearing is free from geared coupling with the motorized friction wheel. The mobile floor support assembly may further include a rigid support structure and a housing enclosing the rigid support structure. The motorized friction wheel may be rotatably mounted to the rigid support structure. The flat bearing may be positioned in a recessed area of the rigid support structure arranged at the base of the mobile floor support assembly. In some examples, the mobile floor support assembly may further include a free wheel driven by the motorized friction wheel. The free wheel may be positioned in the junction near the motorized friction wheel. The motorized friction wheel may enable free rotation of the free wheel. The mobile floor support assembly may include a movement control system including a controller with a processor and computer readable instructions stored on a memory of the controller that when executed cause the controller to swivel the mobile floor support assembly around the swivel axis by operation of the motorized friction wheel. In this way, swiveling is achieved without bulky electromechanical components

The disclosed mobile floor support assembly for a medical imaging assembly offers several advantages. For example, by achieving swiveling without bulky electromechanical components, the mobile floor support assembly enables mounting a C-arm of an X-ray machine at a lower height while maintaining a large open space for imaging. For example, the mobile floor support assembly distributes the mass of the system between the motorized friction wheel and flat bearing. Additionally, the flat bearing, being free from geared coupling with the motorized friction wheel, may be shorter and smaller than conventional chain driven and slewing bearing configurations. In other words, the flat bearing may include a flatter, thinner, and/or narrower profile than chain driven slewing bearings used in comparable systems. Further, in some examples, inclusion of the free wheel may provide additional load bearing support and balance for the overall system, which may enable further reduction of the size of the flat bearing positioned in the base. As a result of these advantages, the footprint of the base of the mobile support assembly may be reduced, including the height and the diameter, which enables mounting a large C-arm at a lower height than comparable C-arm systems. A technical advantage of the herein disclosed mobile floor support assembly includes providing a large C-arm at a comfortable height for medical professionals. As another advantage, the relative simplicity of the flat bearing and motorized friction wheel configuration may be less prone to wear and simpler to manufacture. Overall, the disclosed features produce a C-arm design that is comfortable for medical professionals, a suitable size for a range of patients, and produces high-quality medical images.

FIGS. 2-3, and 5-7 are shown approximately to scale although other relative dimensions may be used. FIGS. 2-3, 5-9 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example. Moreover, the components may be described as they relate to reference axes included in the drawings. As used herein, the term "approximately" is construed to mean plus or minus five percent of the range unless otherwise specified, and the term "substantially equal" means that the elements are sufficiently equal in proportion, size, and/or shape to be considered equal to one of ordinary skilled in the art without being perfectly equal. Features described as "substantially" shaped, e.g., annular, flat, prismatic, circular, etc., means that the features are sufficiently shaped as such to be considered having the shape by one skilled in the art.

Features described as axial may be approximately parallel with an axis referenced unless otherwise specified. Features described as counter-axial may be approximately perpendicular to the axis referenced unless otherwise specified. Features described as radial may circumferentially surround or extend outward from an axis, such as the axis referenced, or a component or feature described prior as being radial to a referenced axis, unless otherwise specified. Features described as longitudinal may be approximately parallel with an axis that is longitudinal. A lateral axis may be normal to the longitudinal axis. Features described as lateral may be approximately parallel with the lateral axis and normal to the longitudinal axis.

Before further discussion of the mobile floor support assembly, an example imaging system in which the assembly may be implemented is shown. Turning now to FIG. 1, a block diagram is shown of an embodiment of an imaging system 10 configured both to acquire original image data and to process the image data for display and/or analysis in accordance with exemplary embodiments. It will be appreciated that various embodiments are applicable to numerous X-ray imaging systems implementing an X-ray tube, such as X-ray radiography (RAD) imaging systems, X-ray mammography imaging systems, fluoroscopic imaging systems, tomographic imaging systems, or CT imaging systems. The following discussion of the imaging system 10 is merely an example of one such implementation and is not intended to be limiting in terms of modality.

As shown in FIG. 1, imaging system 10 includes an X-ray device or X-ray source 12 configured to project a beam of X-rays 14 through an object 16. The object 16 may include a human subject, pieces of baggage, or other objects to be scanned. The X-ray source 12 may be conventional X-ray tubes producing X-rays 14 having a spectrum of energies that range, typically, from thirty keV to two hundred keV. The X-rays 14 pass through the object 16 and, after being attenuated, impinge upon a detector assembly 18. Each detector module in the detector assembly 18 produces an analog electrical signal that represents the intensity of an impinging X-ray beam, and hence the attenuated beam, as it passes through the object 16. In one embodiment, detector assembly 18 is a scintillator based detector assembly, however, it is also envisioned that direct-conversion type detectors (e.g., CdTe, CZT, Si detectors, etc.) may also be implemented.

A processor 20 receives the signals from the detector assembly 18 and generates an image corresponding to the object 16 being scanned. A computer 22 communicates with the processor 20 to enable an operator, using an operator console 24, to control the scanning parameters and to view the generated image. That is, the operator console 24 includes some form of operator interface, such as a keyboard, mouse, voice activated controller, or any other suitable input apparatus that allows an operator to control the imaging system 10 and view the reconstructed image or other data from the computer 22 on a display unit 26. Additionally, the operator console 24 allows an operator to store the generated image in a storage device 28 that may include hard drives, floppy discs, compact discs, etc. The operator may also use the operator console 24 to provide commands and instructions to the computer 22 for controlling a source controller 30 that provides power and timing signals to the X-ray source 12.

An axis system 201 including an x-axis, a y-axis, and a z-axis, is included in FIG. 2 and the figures thereafter for comparison between the figures. The y-axis may be a vertical axis (e.g., parallel to a gravitational axis), the x-axis may be a longitudinal axis, and the z-axis may be a lateral axis. However, the axes may have other orientations in other examples.

Turning to FIG. 2, it illustrates an example embodiment of the imaging system 10, an imaging system 200. In particular, FIG. 2 shows a side view of an X-ray machine 202 including a mobile floor support assembly 208 supporting a medical imaging assembly 204. The medical imaging assembly 204 includes an arm 206, an X-ray tube 214 mounted to the arm 206, and an X-ray detector, hereinafter a detector 216, mounted to the arm 206 opposing the X-ray tube 214. The mobile floor support assembly 208 includes a base 210 and a vertical member 212. The vertical member 212 holds the medical imaging assembly 204 above the base 210. The mobile floor support assembly 208 rests on a floor 236 of an examination room or surgical ward, shown schematically as box 238.

As shown in FIG. 2, the arm 206 may comprise a C-shape. In some examples, the arm 206 may be referred to as a C-arm and the medical imaging assembly 204 may be referred to as a C-arm gantry. The detector 216 is coupled to the arm 206 opposite the X-ray tube 214 and in a direction of emission, indicated by dashed arrow 250. The X-ray tube 214 and the detector 216 are mounted at opposing ends of the arm 206, so that X-rays emitted by the X-ray tube, are incidental to and detected by the detector 216. The X-rays emitted by the X-ray tube 214 may be parallel or collinear with the dashed arrow 250, and the dashed arrow 250 shows a path the X-rays emitted via the X-ray tube 214 may take. The arm 206 may be formed from two C-shaped components including an inner C-shape 240 and an outer C-shape 242. The detector 216 and the X-ray tube 214 may be mounted to the inner C-shape 240. The outer C-shape 242 may house cables and other electrical components.

The mobile floor support assembly 208 may comprise an L-shape. In some examples, the mobile floor support assembly 208 may be referred to as an L-arm. The arm 206 is mounted to the mobile floor support assembly 208 centered on a vertical plane 234 that divides the X-ray machine 202 laterally into substantially equal halves, meaning that the halves would be understood to be equal by a person skilled in the art without being perfectly equal. In some examples, such an arrangement is referred to as a centered C-arc design, or centered C-arc technology, which contrasts with eccentric or offset designs, where the C-arm gantry is offset from the L-arm. The arm 206 extends longitudinally from the vertical member 212 of mobile floor support assembly 208 (e.g., along the x-axis). A cross member 218 rotatably joins the arm 206 and the vertical member 212. The cross member 218 may include a plurality of electromechanical components that control rotation of the arm 206 relative to the mobile floor support assembly 208. The mobile floor support assembly 208 may include a plurality of electromechanical components that control movement of the X-ray machine 202 on the floor 236, such as swiveling or pivoting. The X-ray machine 202 includes a housing 220 that encloses a rigid support structure and the electromechanical components, which are described in more detail below with reference to FIGS. 4-5.

The imaging system 200 may include a patient table 224 supported by a table base 226. The table base 226 may be fixed to the floor 236, or may also be a movable base, such as including caster wheels. In practice, a subject may be positioned on the patient table 224 for imaging. A subject, such as a patient's body, may be supported by the patient table 224. X-ray images may be displayed on a display 228. In one example, the display 228 may display images taken and processed by the imaging system 200 as they are taken and during the imaging procedure (e.g., in real-time). Real-time imaging may further allow for utilization of the imaging system 200 in interventional radiology procedures whereby imaging and treatment of a patient (e.g., laparoscopic surgical procedures, in vivo drug delivery, and the like) may be concurrently performed. The patient table 224 may include a first input device 230 and a second input device 232, whereby user commands may be input to adjust one or more elements of the imaging system 200. For example, the first input device 230 may include a touchpad or a tablet and the second input device 232 may include a joystick. However, other input devices are possible. In one example, a user may input a desired position of one or more of the X-ray machine 202, the patient table 224, the medical imaging assembly 204, the arm 206, or other element of the imaging system 200 to one or both of the first input device 230 and the second input device 232, and in response, the system controller (e.g., computer 22 of FIG. 1) may generate a control signal to adjust the element based on the desired position.

FIG. 3 is a diagram 300 illustrating spatial considerations related to centered C-arc imaging technology, as implemented by the X-ray machine 202. Elements introduced with reference to FIG. 2 are numbered similarly and not reintroduced.

The X-ray machine 202 includes a swivel axis 304 and a central axis 306. The X-ray machine 202 may swivel (or pivot) around the swivel axis 304. In one example, the X-ray machine 202 may be configured to swivel at least 360° around the swivel axis 304. The central axis 306 may divide the arm 206 vertically into substantially equal halves, meaning that the halves would be understood to be equal by a person skilled in the art without being perfectly equal. The arm 206 may rotate (or pivot) about the central axis 306 to adjust the X-ray tube 214 and the detector 216 through a plurality of positions (e.g., at least switch vertical positions, top and bottom, between the detector and X-ray source). In the rotational orientation of the arm 206 shown in FIG. 3, the X-ray tube 214 and the detector 216 are positioned on opposite ends of the arm 206 along the swivel axis 304, where the detector 216 and X-ray tube 214 have surfaces normal to and centered about the swivel axis 304. As an example, the arm 206 may be configured to rotate at least 180 degrees in each direction around the central axis 306. An intersecting axis 320 extends through the intersection of the swivel axis 304 and the central axis 306 parallel with the Z-axis. The arm 206 may rotate around the intersecting axis 320 (e.g., around Z) following the C-shape of the arm. In one example, the arm 206 may rotate around the intersecting axis 320 (e.g., around Z) ± 100°. Other movement of the X-ray machine 202 may include a detector lift, which raises and lowers the detector 216 along a central axis with the X-ray tube 214 (e.g., coaxial with the swivel axis 304 in the orientation shown by FIG. 3), and rotation of detector and collimator blades around the detector-X-ray tube central axis.

A three dimensional opening 308 surrounds the central axis 306. The three dimensional opening 308 extends a vertical distance from a first point 314 aligning with the X-ray tube 214 and a second point 316 aligning with the detector 216 of the arm 206. In some examples, the three dimensional opening 308 may be referred to as a three dimensional tunnel. During an imaging operation, a portion of a subject, such as a portion of a patient's body on patient table 224 in FIG. 2, placed in the three dimensional opening 308 may be irradiated with radiation from the X-ray tube 214. A vertical distance from the floor 236 to the central axis 306 of the medical imaging assembly 204 may be defined as an iso-center height 310. During the imaging operation, a medical professional may position or adjust the patient on the patient table within the three dimensional opening 308 at approximately the iso-center height 310. The radiation may penetrate the portion of the patient's body being irradiated, and travel to the detector 216 where the radiation is captured. By penetrating the portion of the patient's body placed between the X-ray tube 214 and detector 216, an image of the patient's body is obtained and relayed to the display (e.g., display 228 in FIG. 2) where the image is displayed or stored and retrieved later.

Dimensions of the three dimensional opening 308 and the iso-center height 310 may affect the medical professional and outcomes of the medical imaging procedure. For example, a three dimensional opening providing a large open space may be preferred for accommodating a larger subject, such as a larger patient, as well as providing high quality images of internal organs such as the liver. Too high an iso-center height may cause discomfort for the medical professional during examinations. Due to the centered c-arc design with the vertical member 212 holding the arm 206 above the base 210, the iso-center height 310 may be constrained by the height of the base 210, and the electromechanical components enclosed therein, which in turn may constrain the size of the arm 206, including the three dimensional opening 308. A vertical distance from the floor 236 to a point 318 aligning with a bottom of the arm 206 may be defined as a floor height 312. As disclosed herein, the imaging system 200 achieves a comfortable iso-center height and a three dimensional opening providing a large open space by reducing the floor height 312. The approach for reducing the floor height 312 is described below with reference to FIG. 4.

FIG. 4 shows a block diagram illustrating an example of a movement control system 400 for a medical imaging device which achieves a reduced floor height. The movement control system 400 may be included in the imaging system 200 described above with reference to FIGS. 2-3.

The movement control system includes a controller 402 having a processor 404 and a memory 406. The memory 406 may be a non-transitory storage medium including computer-readable instructions that when executed by the controller 402 cause the processor 404 to control movement of the imaging system 200. The processor 404 receives signals from one or more sensors 408 and an operator interface 420, and based on the instructions stored on the memory 406, generates a control signal to a motorized friction wheel comprising a motor 410 and a friction wheel 412, where the friction wheel 412 may be driven by the motor 410. The movement control system 400 may control a swivel movement 416, or pivoting, around a swivel axis 414, which may be similar to the swivel axis 304 in FIG. 3.

In one example, the movement control system 400 may control position, speed, and acceleration of the imaging system in real time based on input from the operator interface 420. In one example, the movement control system 400 may comprise a movement control module that receives a current position of the medical imaging device and a desired position of the medical imaging device, determines a path to move based on the current position and desired position, and generates a control signal to the motor 410 based on the path. Operation of the motor 410 rotates the friction wheel 412 about the swivel axis 414. The movement control system may adapt rotation of the friction wheel 412 based on the angle by operating the motor 410, and further depending on a gear ratio and wheel diameter of the friction wheel 412. In one example, the current position and the desired position may each be defined as an angle relative to a head-toe position of the patient. In some examples, the head-toe position of the patient may be referred to as a table zero rotation. In one example, one or both of the head-toe position of the patient, and the angle of the medical imaging device relative to the head-toe position of the patient may be stored in memory 406 during an idle phase or threshold period at rest. In one example, the control signal may include one or more of a direction of rotation, a speed of rotation, and an acceleration about the swivel axis 414. In other examples, additionally, or alternatively, the control signal may include an (X, Z) coordinate that is determined based on the current position and the desired position. In other examples, the movement control system may control simpler movements such as on/off and clockwise/counterclockwise rotation of the motor 410.

An operator communicates with the processor 404, using the operator interface 420, to control the movement and position the medical imaging device. For example, the operator interface 420 may receive instructions, commands, or other signals indicating a desired position of the medical imaging device from the operator via one or more of a keyboard, mouse, voice activated controller, or any other suitable input apparatus that allows an operator to control the medical imaging device. Examples of the operator interface 420 include the first input device 230 and the second input device 232 described with reference to FIG. 2. In one example, the operator interface 420 may be spaced away from the medical imaging device. For example, as is shown in FIG. 2, the operator interface 420 may be located on a patient table. In other examples, the operator interface 420 may be located on the mobile floor support assembly.

In one example, the processor 404 and the operator interface 420 are connected through a communications bus 418. The communication between the processor 404 and the operator interface 420 set at a distance may be obtained by means of a wireless link. The wireless link may be of any type. For example, the wireless link may be an infrared, ultrasonic, or radiofrequency link, based, for example, on an industrial standard or a proprietary standard, the wireless link may be obtained in a frequency band associated with a protocol such as Wi-Fi, Bluetooth, or others. In other examples, communication between the operator interface 420 and the processor 404 may also be obtained through a wire link.

In one example, the one or more sensors 408 may include one or more of absolute encoders, Hall effect sensors, or potentiometers for detecting the speed, position, angular position, and direction of the imaging system. For example, the one or more sensors 408 may detect the position of the imaging system relative to the head-toe position of the patient. Absolute encoder or other position sensor data may be stored with image data in an X-ray data DICOM (Digital Imaging and Communication in Medicine) file. The encoder or other sensor data may also be displayed on a display screen in a control room as well as in the operating room. In some examples, additionally, or alternatively, the one or more sensors 408 may include an optical position sensor, which may be used for precision measurement of the current position of the X-ray machine, and may also be used to measure an angle or an angular speed about the swivel axis 414. In another example, the one or more sensors 408 may include a force sensor, which may be used to estimate a desired position of the medical imaging device based on an amount of force and direction of force applied to the mobile medical imaging device.

In some examples, the movement control system 400 may similarly control a rotation 428 of a C-arm 424 about a central axis 426, which may be similar to the arm 206 and the central axis 306 described with reference to FIG. 2 and FIG. 3, respectively. For example, the processor 404 may receive signals from the one or more sensors 408 and the operator interface 420, and based on the instructions stored on the memory 406, generate a control signal to a rotatable joint 422 controlling a position of the C-arm 424 coupled to the rotatable joint 422. For example, the movement control system 400 may receive a current position and a desired position of the C-arm 424, determine a path to move based on the current position and desired position, and generate a control signal to the rotatable joint 422 based on the path. For example, the current position and the desired position may each be angles relative to a head-toe position of a patient. The control signal may include one or more of a direction of rotation, a speed of rotation, and a coordinate location about the central axis 426.

FIG. 5 shows an underside view 500 of a rigid support structure 501 for an X-ray machine. In one example, the rigid support structure 501 compactly supports swivel movement of the X-ray machine 202 to enable the large three dimensional opening 308 at a comfortable iso-center height 310, such as described with reference to FIG. 3. An intersection of a first axis 502 and a second axis 504 indicates a point along the swivel axis 304, such as described with reference to FIG. 3. The rigid support structure 501 may swivel around the swivel axis 304. Elements introduced with reference to FIG. 2 and FIG. 5 are numbered similarly and not reintroduced.

The rigid support structure 501 includes a base support structure 510 and a vertical support structure 511. The base support structure 510 and the vertical support structure 511 meet at a junction 514. In one example, the rigid support structure 501 supports the mobile floor support assembly 208 and is enclosed by the housing 220 (see FIG. 2 and FIG. 6). The rigid support structure 501 comprises a plurality of sidewalls 520 which may be of a similar wall depth, an exterior surface 522, and an interior surface 524.

The base support structure 510 may include a narrow portion 542 and a circular portion 540. The circular portion 540 may include a recessed area 518 encircling a raised area 516. The recessed area 518 may be a substantially annular recessed area and the raised area 516 may be a substantially circular raised area. The recessed area 518 may be defined by the interior surface 524 of the circular portion 540, an opening on a floor-facing surface 544, and the interior surface 524 of the raised area 516. A flat bearing 506 positioned in the circular portion 540 of the base support structure 510 supports movement of the rigid support structure 501 around the swivel axis 304. The flat bearing 506 includes a substantially annular shape, including an inner cylindrical surface 554, an outer cylindrical surface 556, an outer bearing surface 546, and an opposing, inner bearing surface (not shown). The flat bearing 506 may be seated in the recessed area 518 with the inner cylindrical surface 554 abutting the raised area 516. When oriented for use, the outer bearing surface 546 of the flat bearing 506 may make contact with the floor (e.g., floor 236 in FIG. 2). The raised area 516 may face but not touch the floor. The flat bearing 506 may include a band 528 positioned around the outer cylindrical surface 556 of the flat bearing 506.

The narrow portion 542 may be a substantially prismatic narrow portion. The narrow portion 542 may be interposed between the circular portion 540 and the vertical support structure 511. The narrow portion 542 may include four perpendicularly arranged sidewalls of the plurality of sidewalls 520. An opening 548 on the floor-facing surface 544 may be arranged at the junction 514 between the vertical support structure 511 and the narrow portion 542 of the base support structure 510. A motorized friction wheel 508 may be positioned at the junction 514. The motorized friction wheel 508 includes a motor 536. In one example, the motorized friction wheel 508 is rotatably mounted to an interior sidewall 512 of the narrow portion 542 of the base support structure 510.

In operation, control of the motorized friction wheel 508 enables the rigid support structure 501 to swivelingly move around the swivel axis 304 in the directions indicated by double headed arrow 526. For example, operation of the motorized friction wheel 508 may generate swivel in a first direction around the swivel axis 304 or an opposing, second direction. The motor 536 of the motorized friction wheel 508 may be operatively coupled to a movement control system, such as the movement control system 400 described with reference to FIG. 4. In response to operator input and one or more sensor signals indicating a current position of the X-ray machine 202, the movement control system may generate a control signal to adjust operation of the motor 536. For example, the motor 536 may rotate in a direction and/or speed based on the control signal. The motor 536 may include sensors 537 (shown schematically), such as absolute encoders or Hall sensors, and a secondary sensor for redundancy. The flat bearing 506 is free from geared coupling with the motorized friction wheel 508 (e.g., there is no chain transmitting torque from the motorized friction wheel 508 to the flat bearing 506). The rigid support structure 501 pivots or swivels around the swivel axis 304 seated on and supported by the flat bearing 506.

The underside view 500 further depicts an arm support structure 538. In one example, the arm support structure 538 supports the arm 206 and is enclosed by the housing 220 (see FIG. 2 and FIG. 6). The arm support structure 538 is rotatably coupled to the vertical support structure 511 of the rigid support structure 501 via a rotatable joint assembly 534. The rotatable joint assembly 534 may comprise a rotatable joint 530 and a grip assembly 532. The arm support structure 538 may include a track 552. The grip assembly 532 may grasp the track 552 to hold the arm support structure 538 above the base support structure 510. Operation of the rotatable joint assembly 534 enables the arm support structure 538 to rotate around an axis 550, which may be similar to the central axis 306 in FIG. 3. Further, operation of the rotatable joint assembly 534 enables the arm support structure 538 to rotate around an axis parallel with the Z-axis (e.g., the intersecting axis 320 in FIG. 3) following the C-shape of the arm, as indicated by double-headed arrow 558. In one example, the rotatable joint 530 of the rotatable joint assembly 534 may be operatively coupled to the movement control system 400. In response to operator input and one or more sensor signals indicating a position of the X-ray machine 202, the movement control system may generate a control signal to adjust operation of the rotatable joint 530, thereby rotating the arm support structure 538 to a desired position around one or both of the axis 550 and the double-headed arrow 558. In other examples, the arm support structure 538 may be controlled to rotate around a dual swivel, multiple axes, or other configurations. For example, some configurations may include a second swivel axis that is configured to enable a vertical member of an L-arm to pivot separately, and in addition to, a base member of the L-arm.

FIG. 6 shows a view 600 depicting a portion of the X-ray machine 202 with the housing 220 and a sidewall of the rigid support structure 501 partially cutaway. The view 600 shows the motorized friction wheel 508 and flat bearing 506 supporting the mobile floor support assembly 208 and the arm 206 mounted thereon. Elements introduced with reference to FIGS. 2-3, and 5 are numbered similarly and not reintroduced.

The view 600 shows the motorized friction wheel 508 positioned at the junction 514 of the of the rigid support structure 501. The rigid support structure 501 includes a plurality of vertically arranged sidewalls 604 and a plurality of horizontally arranged sidewalls 606. The junction 514 includes a first sloped sidewall 608. The base support structure 510 includes a second sloped sidewall 610. In one example, the motor 536 of the motorized friction wheel 508 is rotatably mounted to one of the vertically arranged sidewalls 604. The motorized friction wheel 508 rests on the floor 236, movably supporting the X-ray machine 202.

The flat bearing 506 is positioned in the recessed area 518 of the base support structure 510, surrounding the raised area 516 and supporting the base 210. The flat bearing 506 rests on the floor 236, movably supporting the X-ray machine 202. A bearing height 602 of the flat bearing 506 is indicated. The bearing height 602 of the flat bearing 506 is reduced over comparable centered C-arc imaging systems which commonly include bearings that may be at least twice the bearing height 602 of the flat bearing 506. As a result, a base height 612 of the base 210 may be reduced.

FIG. 7 shows a diagram 700 depicting an X-ray machine of the present disclosure. The diagram 700 shows the X-ray machine 202 including dimensions that may result from the disclosed mobile floor support assembly including the motorized friction wheel for controlling swivel movement around the swivel axis and the flat bearing supporting the swivel axis. Elements introduced with reference to FIGS. 2-3, 5-6 are numbered similarly and not reintroduced.

The X-ray machine 202 includes the central axis 306, the three dimensional opening 308 surrounding the central axis 306, the floor height 312 extending from the floor 236 to a bottom of the arm 206 relative to the central axis 306, a clearance height 716 extending from a top of the base 210 to the bottom of the arm 206 relative to the central axis 306, the iso-center height 310 extending from the central axis 306 to the floor 236, and a rotational axis 714 of the motorized friction wheel 508. The dimensions of the X-ray machine may provide a large open space for imaging at a lower height than comparable C-arm imaging systems using centered C-arc technology. For example, the three dimensional opening 308 as a first ratio of the iso-center height 310 may include a first threshold ratio range of 5.5:7 to 6:7. The floor height 312 as a second ratio of the three dimensional opening 308 may include a second threshold ratio range of 1:5.5 to 1:6. The clearance height 716 as a third ratio of the floor height 312 may include a third threshold ratio range of 1:2 to 1:3. As a result, an operator 710 may operate the X-ray machine 202 comfortably without sacrificing performance or capacity.

C-arm interventional radiology systems, such as the X-ray machine 202, may include design features that balance a tilt moment of the system. For example, the X-ray machine 202 includes center of gravity 712 with a tilt moment comprising a large downward force 702 and a perpendicular force 704. The disclosed approach balances the tilt moment between a first counter force 706 of the flat bearing 506 and a second counter force 708 of the motorized friction wheel 508 (shown schematically). In contrast with comparable C-arc imaging systems, which commonly include a very large and stiff bearing to support the tilt moment, balancing the X-ray machine 202 with the first counter force 706 and the second counter force 708 allows for bearing height 602 reduction by at least 50%. For example, the bearing height 602 may include a threshold height range of 2 cm to 4 cm compared to existing designs which may be in the range of 7 cm to 10 cm.

In some examples, to increase distribution of the tilt moment and balance the weight of the system, the mobile floor support assembly 208 may include a free wheel 720. An example of the free wheel 720 is described below with reference to FIG. 8. The free wheel 720 may add a third counter force 722, allowing further size reduction of the flat bearing 506 and overall footprint of the base 210. For example, a bearing radius 718 of the flat bearing 506 may be reduced in addition to the bearing height 602. In one example, the bearing radius 718 may be reduced by 100-150 mm compared to existing designs. As a result, the operator 710 may perform examinations in a comfortable posture while having more room to place their body or portions of their body, such as their feet, when next to the system.

FIG. 8 shows a first diagram of a wheel assembly 800. The wheel assembly 800 may counter a tilt moment of an X-ray device, such as the tilt moment of the X-ray machine 202 described above with reference to FIG. 7.

The wheel assembly 800 includes a friction wheel 802 and a free wheel 804 driven by the friction wheel 802. The free wheel 804 may be positioned near the friction wheel 802 in an example of the mobile floor support assembly of the present disclosure. In one example, the free wheel 804 may be positioned in the junction 514 near the motorized friction wheel 508 described with reference to FIG. 5. In one example, the free wheel 804 and the friction wheel 802 may each rotatably mount to a wall of a rigid support structure, such as the rigid support structure 501 described with reference to FIG. 5. In one example, the free wheel 804 is free from directly coupling with the friction wheel 802.

The friction wheel 802 may include a first longitudinal axis 806, a first vertical axis 808, and a first rotational axis 810 at an intersection of the first longitudinal axis 806 and the first vertical axis 808. The free wheel 804 may include a second longitudinal axis 812, a second vertical axis 814, and a second rotational axis 816 at an intersection of the second longitudinal axis 812 and the second vertical axis 814. The friction wheel 802 rotates around the first rotational axis 810, generating movement of the friction wheel 802 relative to a floor 818. In one example, rotation of the friction wheel 802 enables free rotation of the free wheel 804 around the second rotational axis 816.

As noted above, replacing a conventional chain transmission and slewing bearing with the friction wheel 802 and swivel bearing (e.g., flat bearing 506 in FIG. 5) reduces tilting torque and load on the bearing. Inclusion of the free wheel 804 further distributes the load, enabling use of a shorter and narrower bearing and reducing the overall packaging space and footprint of the base of the X-ray machine. In one example, the friction wheel 802 may be mounted variously to provide controlled effort. For example, in a wheel system, the ability to perform a motion can be described by the equation F=TotalMass**a*, where the F represents the force provided by the torque of the wheel to perform the movement. The force is applied in the floor. The friction force is given by Friction=*u**MassUnderTheTractionWheel with u the friction coefficient. The maximum force that may be applied to the floor is limited to the friction force. If the applied force exceeds the friction force, the wheel will spin freely. The concept also applies to external forces, where the system will slide if the force is greater than the friction force. By incorporating the free wheel and the traction wheel with controlled effort (e.g., the mass affecting the traction wheel), the system may control the maximum effort provided by the traction wheel, or the value in which the system will move due to an external effort. One approach for controlling effort may include mounting the motorized friction wheel using springs, another may include using the weight of the motorized friction wheel assembly or by using gas springs.

FIG. 9 shows a diagram 900 schematically depicting several possible configurations for a motorized friction wheel and a free that may be positioned in an X-ray machine of the present disclosure, such as the X-ray machine 202 in FIG. 2. The diagram 900 includes a mobile floor support assembly 902 supported by a motorized friction wheel 904, a free wheel 906, and a flat bearing 908.

The motorized friction wheel 904 and the free wheel 906 may be positioned variously, with respect to the mobile floor support assembly 902. Example configurations include a first configuration 920, a second configuration 930, and a third configuration 940. In each configuration, an axis of rotation of the motorized friction wheel 904 and the free wheel 906 coincide with a bearing axis of rotation (or swivel axis). For example, the first configuration 920 may include the free wheel 906 interposed between the motorized friction wheel 904 and the bearing 908. The second configuration 930 may include the motorized friction wheel 904 interposed between the free wheel 906 and the bearing 908. The third configuration 940 may include the motorized friction wheel 904 and the free wheel 906 positioned side-by-side. In the first configuration 920 and the second configuration 930, the free wheel 906 and the motorized friction wheel 904 share a first axis of rotation 910, which intersects with a bearing axis of rotation 912, shown as a filled circle. In the third configuration, the motorized friction wheel 904 rotates on a second axis of rotation 914 and the free wheel 906 rotates on a third axis of rotation 914, where the second axis of rotation 914 and the third axis of rotation 916 each intersect with the bearing axis of rotation 912. In the configurations, rotation of the motorized friction wheel 904 around the first axis of rotation 910 or the second axis of rotation 914 drives rotation of the mobile floor support assembly 902 around the bearing axis of rotation 912.

FIG. 10 shows a flow chart of an example method 1000 for controlling movement of an X-ray machine. The X-ray machine may be the X-ray machine 202 of FIG. 2, for example. The method 1000 may be executed by a movement control system including a controller, such as the controller 402 of the movement control system 400 of FIG. 4, according to instructions stored in a memory of the controller (e.g., the memory 406 of FIG. 4) and in conjunction with one or more inputs, such as inputs received from one or more sensors (e.g., sensors of the plurality of sensors 408 of FIG. 4) or as inputs received from an operator via an operator interface (e.g., operator interface 420 of FIG. 4).

At 1002, the method 1000 may include determining whether a position change of the X-ray machine is indicated. In one example, a position change may be indicated based on the controller receiving a control signal from an operator interface of the X-ray machine. For example, an operator may input a command indicating a desired position to one of the first input device 230 and the second input device 232 in FIG. 2. In one example, a command may include an anatomical command such as a desired position of a patient. In another example, an indication of a position change may be indicated based on the controller receiving a control signal from a force sensor of the X-ray machine. In response to no indication of a position change, the method 1000 may include monitoring for operator input. In response to determining an indication of a position change, the method may continue to 1004.

At 1004, the method 1000 may include determining a current position of the X-ray machine. In one example, the current position of the X-ray machine may include a first current position, which may be defined by an angle of the X-ray machine relative to a head-toe position of a patient. As described above with respect to FIG. 4, the movement control system may include sensors for detecting a position of the X-ray machine. For example, the sensors may include one or more absolute encoders, Hall sensors, or other sensors for detecting a position of the X-ray machine. In one example, in response to determining the position change indication, the method may include obtaining the current position from the absolute encoder or other position sensor. In another example, additionally, or alternatively, the current position of the X-ray machine may be stored in the memory of the controller responsive to a threshold idle period or rest period. For example, the threshold idle period may include a non-zero, positive value threshold period at rest where no movement indication is received, such as 5 seconds. In such an example, in response to determining the position change indication, the position of the X-ray machine may be retrieved from the memory of the controller.

At 1006, the method 1000 may include determining a desired position of the X-ray machine. In one example, the desired position of the X-ray machine may include a first desired position, which may be defined by a desired angle of the base of X-ray machine relative to the head-toe position of the patient. In one example, the desired position may be indicated via the operator interface, such as via one or both of the first input device 230 and the second input device 232. In another example, the desired position of the X-ray machine may be estimated by one or more force sensors detecting an amount of force and direction of force applied to the X-ray machine.

At 1008, the method 1000 may include determining a path to the desired angle. For example, the path may include one or more of a degree of rotation around the swivel axis, a coordinate location, a distance, a direction, or other variables. In one example, the path may include a first path, the first path defined by movement relative to the swivel axis.

At 1010, the method 1000 may include generating a control signal to the motorized friction wheel based on the path. In one example, the control signal may include a pulse signal width, the width determined based on the path. For example, the control signal may adjust one or both of the rotational speed of the motorized friction wheel and the direction of rotation. In one example, the control signal may include a first control signal, the first control signal generated for controlling the motorized friction wheel.

The method 1000 may then return. For example, the method 1000 may be repeated at throughout the X-ray machine operation.

In another example, the method 1000 may be executed for controlling movement of a C-arm of the disclosed X-ray machine. For example, the method 1000 may be carried out to control rotation of the C-arm around the central axis 306 of FIG. 3. For example, the method 1000 may include determining whether a position change of the C-arm is indicated. In response to determining a position change of the C-arm is indicated, the method 1000 may include determining a second current position of the medical imaging assembly. In one example, the second current position may be defined by the angle of the C-arm relative to the head-toe position of the patient. The method 1000 may include determining a second desired position of the medical imaging assembly. In one example, the second desired positon may be defined by the desired angle of the C-arm relative to the head-toe position of the patient. The method 1000 may include determining a second path to the second desired position. The second path may be defined by movement relative to the central axis. For example, the second path may include one or more of a degree of rotation around the central axis, a coordinate location, a distance, a direction, or other variables. The method 1000 may include generating a second control signal to rotate the medical imaging assembly based on the second path. In one example, the second control signal may be generated for controlling a rotatable joint assembly, such as the rotatable joint assembly 534 in FIG. 5. In one example, the second control signal may include a pulse signal width, the width determined based on the second path. The method 1000 describes separate controls for moving the X-ray machine around the central axis and the swivel axis; however, it may be understood that the method 1000 may include combined control of multiple axis. In general, the X-ray machine may be controlled based on anatomical commands, and then swivel and C-arm rotation are computed with the controller to generate the movement in the three-dimensional space. For example, an operator may input an anatomical command, and based on the anatomical command, the controller may adjust one or more of the motorized friction wheel, the rotatable joint assembly, the detector lift, and blades of the detector and/or collimator.

In this way, swiveling movement is achieved for an X-ray machine without bulky electromechanical components. As a result, by reducing the overall footprint of the mobile floor support assembly, a large C-arm may be mounted to the mobile floor support assembly at a lower iso-center height than comparable C-arm systems. The technical effect of the disclosed mobile floor support assembly and X-ray machine comprising the mobile floor support assembly is providing a large three dimensional tunnel at a comfortable working height.

The disclosure also provides support for a mobile floor support assembly for a medical imaging assembly, comprising: a base configured to swivel on a swivel axis, a vertical member coupled to the base, the vertical member configured to hold the medical imaging assembly above the base, a flat bearing positioned in the base, the flat bearing supporting the swivel axis, and a motorized friction wheel positioned at a junction of the vertical member and the base, wherein the motorized friction wheel controls swivel on the swivel axis. In a first example of the system, the flat bearing is free from geared coupling with the motorized friction wheel. In a second example of the system, optionally including the first example, the system further comprises: a rigid support structure and a housing, the housing enclosing the rigid support structure. In a third example of the system, optionally including one or both of the first and second examples, the motorized friction wheel is rotatably mounted to the rigid support structure. In a fourth example of the system, optionally including one or more or each of the first through third examples, the rigid support structure comprises a substantially annular recessed area, wherein the flat bearing is positioned in the substantially annular recessed area. In a fifth example of the system, optionally including one or more or each of the first through fourth examples, the system further comprises: a free wheel driven by the motorized friction wheel. In a sixth example of the system, optionally including one or more or each of the first through fifth examples, the free wheel is positioned in the junction. In a seventh example of the system, optionally including one or more or each of the first through sixth examples, the motorized friction wheel enables free rotation of the free wheel. In an eighth example of the system, optionally including one or more or each of the first through seventh examples, the base comprises a threshold height range of 2 cm to 4 cm. In a ninth example of the system, optionally including one or more or each of the first through eighth examples, the system further comprises: a controller with a processor and computer readable instructions stored on a memory of the controller that when executed cause the controller to swivel the mobile floor support assembly around the swivel axis by operation of the motorized friction wheel.

The disclosure also provides support for an X-ray machine comprising: a medical imaging assembly comprising an arm, an X-ray tube mounted to the arm, and an X-ray detector mounted to the arm opposing the X-ray tube, a mobile floor support assembly supporting the arm and resting on a floor, the mobile floor support assembly comprising a base configured to swivel about a swivel axis, and a vertical member coupled to the base, the vertical member holding the medical imaging assembly above the base, a central axis dividing the arm vertically in substantially equal halves, the arm configured to rotate about the central axis, a three dimensional opening surrounding the central axis, the three dimensional opening extending from a first point aligning with the X-ray tube to a second point aligning with the X-ray detector, the three dimensional opening providing a large open space, a floor height extending from the floor to a bottom of the arm relative to the central axis, a clearance height extending from a top of the base to the bottom of the arm relative to the central axis, and an iso-center height extending from the central axis to the floor, wherein the three dimensional opening as first a ratio of the iso-center height comprises a threshold ratio range of 5.5:7 to 6:7. In a first example of the system, the floor height as a second ratio of the three dimensional opening comprises a second threshold ratio range of 1:5.5 to 1:6. In a second example of the system, optionally including the first example, the clearance height as a third ratio of the floor height comprises a third threshold ratio range of 1:2 to 1:3. In a third example of the system, optionally including one or both of the first and second examples, the arm is mounted to the mobile floor support assembly centered on a vertical plane that divides the X-ray machine laterally into substantially equal halves. In a fourth example of the system, optionally including one or more or each of the first through third examples, the system further comprises: a flat bearing positioned in the base, the flat bearing supporting the swivel axis, and a motorized friction wheel positioned at a junction of the vertical member and the base, the motorized friction wheel controlling the swivel on the swivel axis. In a fifth example of the system, optionally including one or more or each of the first through fourth examples, the system further comprises: a free wheel positioned in the junction, wherein the motorized friction wheel enables free rotation of the free wheel. In a sixth example of the system, optionally including one or more or each of the first through fifth examples, the system further comprises: a rotatable joint assembly, the rotatable joint assembly rotatably mounting the arm to the mobile floor support assembly.

The disclosure also provides support for a method for an X-ray machine, the X-ray machine comprising a mobile floor support assembly configured to swivel on a swivel axis by operation of a motorized friction wheel, and a medical imaging assembly rotatably mounted to the mobile floor support assembly, the method comprising: determining a first current position of the mobile floor support assembly, wherein the mobile floor support assembly comprises a clearance height and a floor height, wherein the clearance height as a first ratio of the floor height comprises a first threshold ratio range of 1:2 to 1:3, determining a first desired position, determining a first path to the first desired position, and generating a first control signal to the motorized friction wheel based on the first path. In a first example of the method, the first desired position comprises a desired angle relative to a head-toe position of a patient. In a second example of the method, optionally including the first example, the method further comprises:, determining a second current position of the medical imaging assembly, wherein the medical imaging assembly comprises a three dimensional opening and an iso-center height, wherein the three dimensional opening as a second ratio of the iso-center height comprises a second threshold ratio range of 5.5:7 to 6:7, determining a second desired position, determining a second path to the second desired position, and generating a second control signal to rotate the medical imaging assembly based on the second path.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A mobile floor support assembly (208) for a medical imaging assembly (204), comprising:
a base (210) configured to swivel on a swivel axis (304);
a vertical member (212) coupled to the base (210), the vertical member (212) configured to hold the medical imaging assembly (204) above the base (210);
a flat bearing (506) positioned in the base (210), the flat bearing (506) supporting the swivel axis (304); and
a motorized friction wheel (508) positioned at a junction (514) of the vertical member (212) and the base (210),
wherein the motorized friction wheel (508) controls swivel on the swivel axis (304).

2. The mobile floor support assembly (208) of claim 1, wherein the flat bearing (506) is free from geared coupling with the motorized friction wheel (508).

3. The mobile floor support assembly (208) of claim 1, further comprising a rigid support structure (501) and a housing (220), the housing (220) enclosing the rigid support structure (501).

4. The mobile floor support assembly (208) of claim 3, wherein the motorized friction wheel (508) is rotatably mounted to the rigid support structure (501).

5. The mobile floor support assembly (208) of claim 3, wherein the rigid support structure (501) comprises a substantially annular recessed area (518), wherein the flat bearing (506) is positioned in the substantially annular recessed area (518).

6. The mobile floor support assembly (208) of claim 1, further comprising a free wheel (804) driven by the motorized friction wheel (802).

7. The mobile floor support assembly (208) of claim 6, wherein the free wheel (720) is positioned in the junction (514).

8. The mobile floor support assembly (208) of claim 6, wherein the motorized friction wheel (802) enables free rotation of the free wheel (804).

9. The mobile floor support assembly (208) of claim 1, wherein the base (210) comprises a threshold height range of 2 cm to 4 cm.

10. The mobile floor support assembly (208) of claim 1, further comprising a controller (402) with a processor (404) and computer (22) readable instructions stored on a memory (406) of the controller (402) that when executed cause the controller (402) to swivel the mobile floor support assembly (208) around the swivel axis (414) by operation of the motorized friction wheel (412).

11. An X-ray machine (202) comprising:
a medical imaging assembly (204) comprising an arm (206), an X-ray tube (214) mounted to the arm (206), and an X-ray detector (216) mounted to the arm (206) opposing the X-ray tube (214);
a mobile floor support assembly (208) supporting the arm (206) and resting on a floor, the mobile floor support assembly (208) comprising a base (210) configured to swivel about a swivel axis (550, 414, 304), and a vertical member (212) coupled to the base (210), the vertical member (212) holding the medical imaging assembly (204) above the base (210);
a central axis (306) dividing the arm (206) vertically in substantially equal halves, the arm (206) configured to rotate about the central axis (306);
a three dimensional opening (308) surrounding the central axis (306), the three dimensional opening (308) extending from a first point (314) aligning with the X-ray tube (214) to a second point (316) aligning with the X-ray detector (216), the three dimensional opening (308) providing a large (3D) open space;
a floor height (312) extending from the floor to a bottom of the arm (206) relative to the central axis (306);
a clearance height (716) extending from a top of the base (210) to the bottom of the arm (206) relative to the central axis (306); and
an iso-center height (310) extending from the central axis (306) to the floor,
wherein the three dimensional opening (308) as first a ratio of the iso-center height (310) comprises a threshold ratio range of 5.5:7 to 6:7.

12. The X-ray machine (202) of claim 11, wherein the floor height (312) as a second ratio of the three dimensional opening (308) comprises a second threshold ratio range of 1:5.5 to 1:6.

13. The X-ray machine (202) of claim 11, wherein the clearance height (716) as a third ratio of the floor height (312) comprises a third threshold ratio range of 1:2 to 1:3.

14. The X-ray machine (202) of claim 11, wherein the arm (206) is mounted to the mobile floor support assembly (208) centered on a vertical plane (234) that divides the X-ray machine (202) laterally into substantially equal halves.

15. The X-ray machine (202) of claim 11, further comprising a flat bearing (506) positioned in the base (210), the flat bearing (506) supporting the swivel axis (304), and a motorized friction wheel (508) positioned at a junction (514) of the vertical member (212) and the base (210), the motorized friction wheel (508) controlling the swivel on the swivel axis (304).
